# EUROPEAN PATENT APPLICATION

(11) **EP 3 332 771 A1**
(43) Date of publication of application: **13.06.2018**
(21) Application number: 17205656.6
(22) Date of filing: 06.12.2017
(51) Int. Cl.: A61K 9/20, A61K 31/4439

(54) **MULTILAYERED TABLET COMPOSITIONS OF DABIGATRAN**

(30) Priority: 07.12.2016 TR 201617984; 28.12.2016 TR 201619828
(71) Applicant: Sanovel Ilac Sanayi ve Ticaret A.S., 34460 Istanbul (TR)
(72) Inventor: TÜRKYILMAZ, Ali, 34460 Istanbul (TR); PALANTÖKEN, Arzu, 34460 Istanbul (TR); GÜLKOK, Yildiz, 34460 Istanbul (TR); SÖZER BÜRKÜT, Gökce, 34460 Istanbul (TR)
(74) Representative: Sevinç, Erkan

(57) **Abstract**

The present invention relates to a multilayered pharmaceutical composition for oral administration comprising dabigatran etexilate free base or pharmaceutically acceptable salts of dabigatran etexilate and at least one pharmaceutically acceptable excipient.

## Description

### Technical Field

The present invention relates to a multilayered pharmaceutical composition for oral administration comprising dabigatran etexilate free base or pharmaceutically acceptable salts of dabigatran etexilate and at least one pharmaceutically acceptable excipient.

### Background of the Invention

Dabigatran etexilate (Formula 1), which is already known from WO 98/37075, is a direct thrombin inhibitor indicated to reduce the risk of stroke and systemic embolism in patients with non-valvular atrial fibrilation. Thrombin is a multifunctional enzyme which converts fibrinogen to fibrin, cross-linking fibrin monomers via activation of factor XIII and augmenting further thrombin production via the activation of factors V and VIII. It also activates platelets, generates anticoagulant activity via activation of protein C and initiates numerous cellular processes.

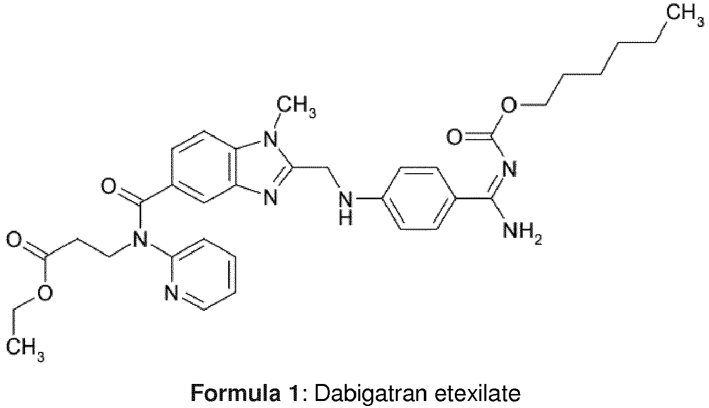

The methane sulphonic acid addition salt of dabigatran etexilate, which is commercially available under the trade name PRADAXA® immediate release capsule (in the strength of 75, 110, 150 mg), is disclosed in EP1870100, wherein also disclosed, pellet formulation of dabigatran etexilate methanesulphonate. This composition is formulated with a core material consisting of organic acid and an active layer which encloses the core. Each PRADAXA® capsule contains the following inactive ingredients: acacia, dimethicone, hypromellose, hydroxypropylcellulose, tartaric acid, carrageenan, potassium chloride, talc, titanium dioxide, and gelatin.

Apart from the methanesulfonate salt of dabigatran etexilate, other acid addition salts of the compound are provided in prior art. For example, WO2012/077136 is directed to the oxalate salt of dabigatran etexilate and besides, its hydrochloride salt is identified in EP1877395.

It is known that the solubility of weakly basic drugs, such as dabigatran and dabigatran etexilate, is pH-dependent and may be increased by the provision of an acidic environment. Therefore, weakly basic drugs are formulated with an acidic excipient.

Patent application EP1658056 discloses a conventional tablet formulation comprising dabigatran etexilate, organic acid with a solubility in water of > 1 g / 250 ml at 20°C together with conventional excipients and fillers. But in the patent application EP1658056, there is no insulating layer between the active agent and the organic acid.

In prior art, most of formulations are in the form of capsules. But it is known that capsules cost more than tablets and have significant space and potency limitations since their powdered contents cannot be compressed to a significant degree. Since capsules are not airtight, their shelf-life is shorter than tablets.

The pellet formulation used in the marketed product Pradaxa® is produced by a difficult production method which has a lot of production steps causing the process being longer than others. For pellet processing specially designed production equipment has to be used.

Thus, there is still a need in the art to develop stable and bioavailable pharmaceutical formulations comprising dabigatran etexilate free base or pharmaceutically acceptable salts of dabigatran etexilate with a long shelf-life and a pH independent release by a simplified and cost effective process. By this need, the formulation has been developed to overcome the solubility and stability problems of dabigatran etexilate in a safe manner disclosed above.

### Detailed description of the Invention

The main object of the present invention is to provide a multi-layered pharmaceutical composition for oral administration comprising dabigatran etexilate free base or pharmaceutically acceptable salts of dabigatran etexilate and at least one pharmaceutically acceptable excipient.

Multi-layered tablets are known as a novel drug delivery system. Compaction of different granules in the form of various layer in single tablets are called as multi-layered tablets. It generally consists of parallel, clear, coloured, visual distinct layers two to three or more APIs or APIs along with functional or non-functional placebo layers. Multi-layered tablet dosage forms are designed for a variety of reasons such as incompatibility problems between active agents or excipients.

According to main embodiment of the invention, there is an inert layer between active agent layer (first layer) and acidic layer (second layer) to avoid incompatibility problems between them.

According to this embodiment, the multi-layered pharmaceutical composition for oral administration comprises:
a) A first layer comprising dabigatran etexilate free base or pharmaceutically acceptable salts of dabigatran etexilate and at least one pharmaceutically acceptable excipient
b) an inert layer comprising at least one pharmaceutically acceptable excipient
c) a second layer comprising one pharmaceutically acceptable acid or hydrates or acid salts thereof and at least one pharmaceutically acceptable excipient

As used herein, the term "dabigatran etexilate free base" refers to dabigatran etexilate which is free from other forms of the active moiety, especially acid addition salts.

According to one embodiment, dabigatran etexilate free base or pharmaceutically acceptable salts of dabigatran etexilate is present in the first layer in an amount of between 5.00 and 60.00 %, preferably between 15.00 and 50.00 % and more preferably it is 25.00 to 40.00 % by weight of the first layer.

According to another embodiment, dabigatran etexilate free base is present in an amount of between 30 to 350 mg, preferably 50 to 300 mg and more preferably it is 50 to 250 mg.

According to one embodiment of this present invention, wherein said at least one pharmaceutically acceptable excipient for each layer is selected from disintegrants, lubricants, glidants, binders, fillers, colouring agents or mixtures thereof.

According to another embodiment of this present invention, wherein at least one pharmaceutically acceptable excipient in the first layer is disintegrant. During the development study of the present invention, it has been observed that, dissolution and stability problems of the active agent are overcome by using particularly at least two different disintegrants in the first layer in a specific amount.

According to one embodiment, the first layer of the pharmaceutical composition comprises preferably at least two different disintegrants.

According to another embodiment, the amount of total disintegrants in the first layer is at least 40% by weight of the first layer. Preferably the amount of total disintegrants in the first layer is between 40% and 60% by weight of the first layer. More preferably the amount of total disintegrants in the first layer is between 40% and 50% by weight of the first layer.

Suitable disintegrants are selected from a group comprising pregelatinized starch, low-substituted hydroxypropyl cellulose (L-HPC), microcrystalline cellulose, sodium starch glycolate, starch, crospovidone (cross-linked polyvinil pyrrolidone), povidone, poloxomer, cross-linked carboxymethyl cellulose (croscarmellose sodium), sodium carboxymethyl cellulose, calcium carboxymethyl cellulose, carboxymethyl cellulose, docusate sodium, guar gum, polyacryline potassium, sodium alginate, alginic acid, alginates, ion-exchange resins, magnesium aluminium silica, sodium dodesyl sulphate, poloxamer, sodium glycine carbonate, sodium lauryl sulphate or mixtures thereof.

In a preferred embodiment, the disintegrants are selected from microcrystalline cellulose, pregelatinized starch, low-substituted hydroxypropyl cellulose (L-HPC) and sodium starch glycolate.

Suitable lubricants are selected from a group comprising magnesium stearate, sodium stearyl fumarate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium lauryl sulphate or mixtures thereof.

In a preferred embodiment, the lubricant is magnesium stearate.

According to these embodiments, the lubricant is present an amount of between 0.1% and 10.00%, preferably 0.1% and 5.00% and more preferably 0.1% and 3.00% by weight of each layer.

Suitable glidants are selected from a group comprising colloidal silicon dioxide, talc, aluminium silicate or mixtures thereof.
In a preferred embodiment, the glidant is colloidal silicon dioxide.

According to this embodiment, the glidant is present an amount of between 0.1% and 10.00%, 0.5% and 5.00% and more preferably 0.9% and 3.00% by weight of each layer.

Suitable fillers are selected from a group comprising lactose, mannitol, spray-dried mannitol, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate, polyols, dextrose, maltitol or mixtures thereof.

In a preferred embodiment, the filler is lactose in the first layer and microcrystalline cellulose in the inert layer and the second layer.

Suitable binders are selected from the group comprising pregelatinized starch, povidone, copovidone, copolyvidone, polyvinylpyrrolidone, carnauba wax, hydroxypropyl methyl cellulose, pullulan, polymethacrylate, glyceryl behenate, hydroxypropyl cellulose, carboxymethyl cellulose, methyl cellulose, hydroxyethyl cellulose, sodium carboxymethyl cellulose, carboxymethyl cellulose calcium, ethyl cellulose, microcrystalline cellulose, polymetacrylates, polyethylene oxide, polyvinyl alcohol, polycarbophil, polyvinyl acetate and its copolymers, gelatin, starch, xanthan gum, guar gum, alginate, carrageen, kollagen, agar, pectin, hyaluronic acid, carbomer, cellulose acetate phthalate, hydroxypropyl starch, hydroxyethyl methyl cellulose, polaxomer, polyethylene glycol, sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose or mixtures thereof.

In a preferred embodiment, the binders are selected from pregelatinized starch, povidone or mixtures thereof.

According to one embodiment, wherein said one pharmaceutically acceptable acid is selected from tartaric acid, fumaric acid, succinic acid, citric acid, malic acid, glutamic acid and aspartic acid or hydrates or salts thereof.

According to this embodiment, preferably the pharmaceutically acceptable acid is selected from citric acid and tartaric acid.

According to another embodiment, the pharmaceutically acceptable acid is present in the second layer in an amount of between 10% and 90, preferably between 15% and 70%, more preferably between 20% and 40% by weight of the second layer.

According to one embodiment, wherein the weight ratio of dabigatran etexilate free base or pharmaceutically acceptable salt of dabigatran etexilate to pharmaceutically acceptable acid is between 0.5 and 2.0, preferably between 1.0 and 2.0, more preferably between 1.0 and 1.6.

In one embodiment, the inert layer comprises a colouring agent such as yellow iron oxide.

According to this embodiment, colouring agent is present in an amount of between 0.1% and 2.0, preferably between 0.5 and 2.0, more preferably between 0.5 and 1.0 by weight of the inert layer.

Suitable salts of dabigatran etexilate are selected from a group comprising mesylate, maleate, malonate, citrate, tosylate, dabigatran esylate, tartrate, oxalate, camphor sulfonate.

Preferably suitable salts of dabigatran etexilate are selected from maleate, malonate, citrate, tosylate, esylate, tartrate, oxalate, camphor sulfonate.

### Example 1: Multi-layered tablet

| **First Layer** | **(%) amount (w/w)** |
|---|---|
| Dabigatran etexilate free base or pharmaceutically acceptable salts of dabigatran etexilate | 5.00-60.00 |
| Sodium starch glycolate | 0.25-8.00 |
| Microcrystalline celluose | 10.00-70.00 |
| Colloidal silicon dioxide | 0.1-5.00 |
| Starch, pregelatinize | 5.00-30.00 |
| Lactose | 5.00-50.00 |
| Magnesium stearate | 0.1-5.00 |
| **Total (First layer)** | **100** |

| **Inert Layer** | **(%) amount (w/w)** |
|---|---|
| Povidone | 1.00-10.00 |
| Microcrystalline cellulose | 70.00-95.00 |
| Yellow iron oxide | 0.1-2.00 |
| Colloidal silicon dioxide | 0.05-5.00 |
| Magnesium stearate | 0.1-5.00 |
| **Total (Inert Layer)** | **100** |

| **Second Layer** | **(%) amount (w/w)** |
|---|---|
| Tartaric acid | 10.00-90.00 |
| L-HPC | 1.00-20.00 |
| Microcrystalline cellulose | 30.00-70.00 |
| Colloidal silicon dioxide | 0.05-5.00 |
| Magnesium stearate | 0.1-5.00 |
| **Total (Second Layer)** | **100** |

### Example 2: Multi-layered tablet

| **First Layer** | **(%) amount (w/w)** |
|---|---|
| Dabigatran etexilate free base or pharmaceutically acceptable salts of dabigatran etexilate | 25.00-40.00 |
| Sodium starch glycolate | 1.00-2.00 |
| Microcrystalline celluose | 25.00-35.00 |
| Colloidal silicon dioxide | 0.5-2.00 |
| Starch, pregelatinize | 10.00-20.00 |
| Lactose | 15.00-25.00 |
| Magnesium stearate | 0.5-2.00 |
| **Total (First layer)** | **100** |

| **Inert Layer** | **(%) amount (w/w)** |
|---|---|
| Povidone | 5.00-10.00 |
| Microcrystalline cellulose | 90.00-95.00 |
| Yellow iron oxide | 0.5-1.00 |
| Colloidal silicon dioxide | 0.1-1.00 |
| Magnesium stearate | **0.1-1.00** |
| **Total (Inert Layer)** | **100** |

| **Second Layer** | **(%) amount (w/w)** |
|---|---|
| Tartaric acid | 20.00-40.00 |
| L-HPC | 1.00-5.00 |
| Microcrystalline cellulose | 50.00-70.00 |
| Colloidal silicon dioxide | 0.5-5.00 |
| Magnesium stearate | 0.5-2.00 |
| **Total (Second Layer)** | **100** |

The pharmaceutical compositions mentioned above are prepared by following these steps:

### First layer

- Weighing, sieving and mixing sodium starch glycolate, microcrystalline cellulose, colloidal silicon dioxide, pregelatinized starch, lactose.
- Granulating the powder mixture with dabigatran etexilate which is dispersed in water
- Drying in a vacuum oven at 50-55°C.
- Adding sieved magnesium stearate to the mixture and mixing.

### Inert layer

- Weighing povidone, microcrystalline cellulose, colloidal silicon dioxide and yellow iron oxide and mixing them in a mixer.
- Adding magnesium stearate to the mixture and mixing.

### Second layer

- Weighing and mixing tartaric acid, L-HPC, microcrystalline cellulose and colloidal silicon dioxide and mixing them in a mixer.
- Adding magnesium stearate to the mixture and mixing.

Pressing these three mixtures for each layer into trilayer tablets.
Coating these tablets.

### Example 3: Multi-layered tablet

| **First Layer** | **(%) amount (w/w)** |
|---|---|
| Dabigatran etexilate free base or pharmaceutically acceptable salts of dabigatran etexilate | 5.00-60.00 |
| Sodium starch glycolate | 0.25-8.00 |
| Microcrystalline celluose | 10.00-70.00 |
| Colloidal silicon dioxide | 0.1-5.00 |
| Starch, pregelatinize | 5.00-30.00 |
| Lactose | 5.00-50.00 |
| Magnesium stearate | 0.1-5.00 |
| **Total (First layer)** | **100** |

| **Inert Layer** | **(%) amount (w/w)** |
|---|---|
| Starch, pregelatinize | 1.00-10.00 |
| Microcrystalline cellulose | 70.00-95.00 |
| Yellow iron oxide | 0.1-2.00 |
| Colloidal silicon dioxide | 0.05-5.00 |
| Magnesium stearate | 0.1-5.00 |
| **Total (Inert Layer)** | **100** |

| **Second Layer** | **(%) amount (w/w)** |
|---|---|
| Citric acid | 10.00-90.00 |
| Starch, pregelatinize | 1.00-20.00 |
| Microcrystalline cellulose | 30.00-70.00 |
| Colloidal silicon dioxide | 0.05-5.00 |
| Magnesium stearate | 0.1-5.00 |
| **Total (Second Layer)** | **100** |

### Example 4: Multi-layered tablet

| **First Layer** | **(%) amount (w/w)** |
|---|---|
| Dabigatran etexilate free base or pharmaceutically acceptable salts of dabigatran etexilate | 25.00-40.00 |
| Sodium starch glycolate | 1.00-2.00 |
| Microcrystalline celluose | 25.00-35.00 |
| Colloidal silicon dioxide | 0.5-2.00 |
| Starch, pregelatinize | 10.00-20.00 |
| Lactose | 15.00-25.00 |
| Magnesium stearate | 0.5-2.00 |
| **Total (First layer)** | **100** |

| **Inert Layer** | **(%) amount (w/w)** |
|---|---|
| Povidone | 5.00-10.00 |
| Microcrystalline cellulose | 90.00-95.00 |
| Yellow iron oxide | 0.5-1.00 |
| Colloidal silicon dioxide | 0.1-1.00 |
| Magnesium stearate | 0.1-1.00 |
| **Total (Inert Layer)** | **100** |

| **Second Layer** | **(%) amount (w/w)** |
|---|---|
| Citric acid | 20.00-40.00 |
| Starch, pregelatinize | 1.00-5.00 |
| Microcrystalline cellulose | 50.00-70.00 |
| Colloidal silicon dioxide | 0.5-5.00 |
| Magnesium stearate | 0.5-2.00 |
| **Total (Second Layer)** | **100** |

The pharmaceutical compositions mentioned above are prepared by following these steps:

### First layer

- Weighing, sieving and mixing sodium starch glycolate, microcrystalline cellulose, colloidal silicon dioxide, pregelatinized starch, lactose and dabigatran etexilate free base or pharmaceutically acceptable salt of dabigatran etexilate.
- Granulating the powder mixture with water.
- Drying in a vacuum oven at 50-55°C.
- Adding magnesium stearate to the mixture and mixing.

### Inert layer

- Sieving pregelatinized starch, microcrystalline cellulose, colloidal silicon dioxide and yellow iron oxide and mixing them in a mixer.
- Adding magnesium stearate to the mixture and mixing.

### Second layer

- Sieving and mixing citric acid, pregelatinized starch, microcrystalline cellulose and colloidal silicon dioxide and mixing them in a mixer.
- Adding magnesium stearate to the mixture and mixing 1-2 more minutes

Pressing these three mixtures for each layer into trilayer tablets.
Coating these tablets.

## Claims

1. A pharmaceutical composition for oral administration comprising:
a) A first layer comprising dabigatran etexilate free base or pharmaceutically acceptable salts of dabigatran etexilate and at least one pharmaceutically acceptable excipient
b) an inert layer comprising at least one pharmaceutically acceptable excipient
c) a second layer comprising one pharmaceutically acceptable acid or hydrates or acid salts thereof and at least one pharmaceutically acceptable excipient

2. The oral pharmaceutical composition according to claim 1, wherein the amount of dabigatran etexilate free base or pharmaceutically acceptable salts of dabigatran etexilate is 5.00% to 60.00% by weight of the first layer.

3. The pharmaceutical composition according to claim 2, wherein the amount of dabigatran etexilate free base or pharmaceutically acceptable salts of dabigatran etexilate is between 30 to 350 mg.

4. The pharmaceutical composition according to claim 1, wherein said at least one pharmaceutically acceptable excipient for each layer is selected from disintegrants, lubricants, glidants, binders, fillers, colour agents or mixtures thereof.

5. The pharmaceutical composition according to claim 4, wherein the first layer of the pharmaceutical composition comprising preferably at least two different disintegrants.

6. The pharmaceutical composition according to claim 4, wherein the second layer of the pharmaceutical composition comprising at least one disintegrant.

7. The pharmaceutical comporision according to claim 5, wherein the amount of total disintegrants in the first layer is at least 40% by weight of the first layer.

8. The pharmaceutical composition according to claim 6, wherein the disintegrants are selected from a group comprising starch, sodium starch glycolate, microcrystalline cellulose, crospovidone (cross-linked polyvinil pyrrolidone), povidone, poloxomer, cross-linked carboxymethyl cellulose (croscarmellose sodium), low-substituted hydroxypropyl cellulose, pregelatinized starch, sodium carboxymethyl cellulose, calcium carboxymethyl cellulose, carboxymethyl cellulose, docusate sodium, guar gum, polyacryline potassium, sodium alginate, alginic acid, alginates, ion-exchange resins, magnesium aluminium silica, sodium dodesyl sulphate, poloxamer, sodium glycine carbonate, sodium lauryl sulphate or mixtures thereof, preferably the disintegrants are sodium starch glycolate, microcrystalline cellulose, low-substituted hydroxypropyl cellulose, pregelatinized starch or mixtures thereof.

9. The pharmaceutical composition according to claim 1, wherein said one pharmaceutically acceptable acid is selected from tartaric acid, fumaric acid, succinic acid, citric acid, malic acid, glutamic acid and aspartic acid or hydrates or salts thereof.

10. The pharmaceutical composition according to claim 9, wherein the amount of pharmaceutically acceptable acid in the second layer is between 10% and 90, preferably between 15% and 70%, more preferably between 20% and 40% by weight of the second layer.

11. The pharmaceutical composition according to claim 1 or 9, wherein the weight ratio of dabigatran etexilate free base or pharmaceutically acceptable salt of dabigatran etexilate to pharmaceutically acceptable acid is between 0.5 and 2.0, preferably between 1.0 and 2.0, more preferably between 1.0 and 1.6.

12. The pharmaceutical composition according to any of the preceding claims, comprising:
a. 5.00 to 60.00 % dabigatran etexilate free base or pharmaceutically acceptable salts of dabigatran etexilate
b. 0.25 to 8.00 % sodium starch glycolate
c. 10.00 to 70.00 % microcrystalline celluose
d. 0.1 to 5.00 % colloidal silicon dioxide
e. 5.00 to 30.00 % starch, pregelatinize
f. 5.00 to 50.00 % lactose
g. 0.1 to 5.00 % magnesium stearate
in the first layer by weight of the first layer
h. 1.00 to 10.00 % povidone
i. 70.00 to 95.00 % microcrystalline cellulose
j. 0.1 to 2.00 % yellow iron oxide
k. 0.05 to 5.00 % colloidal silicon dioxide
l. 0.1 to 5.00 % magnesium stearate
in the inert layer by weight of the inert layer
m. 10.00 to 90.00 % tartaric acid
n. 1.00 to 20.00 % low-substituted hydroxypropyl cellulose
o. 30.00 to 70.00 % microcrystalline cellulose
p. 0.05 to 5.00 % colloidal silicon dioxide
r 0.1 to 5.00 % magnesium stearate
in the second layer by weight of the second layer

13. The pharmaceutical composition according to any of the preceding claims, comprising:
a. 5.00 to 60.00 % dabigatran etexilate free base or pharmaceutically acceptable salts of dabigatran etexilate
b. 0.25 to 8.00 % sodium starch glycolate
c. 10.00 to 70.00 % microcrystalline celluose
d. 0.1 to 5.00 % colloidal silicon dioxide
e. 5.00 to 30.00 % starch, pregelatinize
f. 5.00 to 50.00 % lactose
g. 0.1 to 5.00 % magnesium stearate
in the first layer by weight of the first layer
h. 1.00 to 10.00 % starch, pregelatinize
i. 70.00 to 95.00 % microcrystalline cellulose
j. 0.1 to 2.00 % yellow iron oxide
k. 0.05 to 5.00 % colloidal silicon dioxide
l. 0.1 to 5.00 % magnesium stearate
in the inert layer by weight of the inert layer
m. 10.00 to 90.00 % citric acid
n. 1.00 to 20.00 % starch, pregelatinize
o. 30.00 to 70.00 % microcrystalline cellulose
p. 0.05 to 5.00 % colloidal silicon dioxide
r. 0.1 to 5.00 % magnesium stearate
in the second layer by weight of the second layer

14. The process for preparation of the pharmaceutical composition according to claim 12, wherein the process comprising the following steps:
a. first layer
i. weighing, sieving and mixing sodium starch glycolate, microcrystalline cellulose, colloidal silicon dioxide, pregelatinized starch, lactose.
ii. granulating the powder mixture with dabigatran etexilate which is dispersed in water
iii. drying in a vacuum oven at 50-55°C.
iv. adding sieved magnesium stearate to the mixture and mixing.
b. inert layer
i. weighing povidone, microcrystalline cellulose, colloidal silicon dioxide and yellow iron oxide and mixing them in a mixer.
ii. adding magnesium stearate to the mixture and mixing.
c. second layer
i. weighing and mixing tartaric acid, L-HPC, microcrystalline cellulose and colloidal silicon dioxide and mixing them in a mixer.
ii. adding magnesium stearate to the mixture and mixing.
d. pressing these three mixtures for each layer into trilayer tablets.
e. coating these tablets.

15. The process for preparation of the pharmaceutical composition according to claim 13, wherein the process comprising the following steps:
a. first layer
i. weighing, sieving and mixing sodium starch glycolate, microcrystalline cellulose, colloidal silicon dioxide, pregelatinized starch, lactose and dabigatran etexilate free base or pharmaceutically acceptable salt of dabigatran etexilate
ii. granulating the powder mixture with water
iii. drying in a vacuum oven at 50-55°C.
iv. adding magnesium stearate to the mixture and mixing.
b. inert layer
i. sieving pregelatinized starch, microcrystalline cellulose, colloidal silicon dioxide and yellow iron oxide and mixing them in a mixer.
ii. adding magnesium stearate to the mixture and mixing.
c. second layer
i. sieving and mixing citric acid, pregelatinized starch, microcrystalline cellulose and colloidal silicon dioxide and mixing them in a mixer.
ii. adding magnesium stearate to the mixture and mixing 1-2 more minutes
d. pressing these three mixtures for each layer into trilayer tablets.
e. coating these tablets.
